# EUROPÄISCHE PATENTSCHRIFT

(11) **EP 4 554 895 B1**
(45) Veröffentlichungstag und Bekanntmachung des Hinweises auf die Patenterteilung: **24.12.2025**
(21) Anmeldenummer: 22757851.5
(22) Anmeldetag: 13.07.2022
(51) Int. Cl.: C01B 33/18, C01B 33/32, C07C 1/32, C01B 33/113

(54) **VERFAHREN ZUR BASENKATALYSIERTEN UMSETZUNG VON SILICONEN MIT HYDROTHERMALEM ODER ÜBERKRITISCHEM WASSER**
PROCESS FOR BASE-CATALYZED REACTION OF SILICONES WITH HYDROTHERMAL OR SUPERCRITICAL WATER
PROCÉDÉ DE RÉACTION CATALYSÉE PAR UNE BASE, DE SILICONES AVEC DE L'EAU HYDROTHERMALE OU SUPERCRITIQUE

(43) Veröffentlichungstag der Anmeldung: 21.05.2025
(73) Patentinhaber: Wacker Chemie AG, 81671 München (DE)
(72) Erfinder: DELLERMANN, Theresa, 81371 Muenchen (DE); SCHMID, Stefan, 80687 Muenchen (DE); WIENKENHOEVER, Niklas, 81545 München (DE)
(74) Vertreter: Wimmer, Andreas
(86) Internationale Anmeldenummer: PCT/EP2022/069625
(87) Internationale Veröffentlichungsnummer: WO 2024/012670

(56) Entgegenhaltungen:
- WO-A1-2022/214176
- CN-A- 111 498 854

## Beschreibung

Die Erfindung betrifft ein basenkatalysiertes Verfahren zur Umsetzung von Silicon mit hydrothermalem oder überkritischem Wasser.

CN 111 498 854 offenbart ein Verfahren zur Herstellung von Siliziumdioxid durch Reaktion von Siliziumpulver mit superkritischem Wasser in basischer Umgebung, wobei die Temperatur 374°C bis 450°C beträgt. Die Reaktionsdauer beträgt 0,1 bis 4 Stunden. Der Vorteil des Verfahrens liegt u.a. darin, dass ein Produkt mit hoher Reinheit erhalten wird.

Werden ausgehärtete Siliconmassen nach dem Ende des Lebenszyklus entsorgt, landen sie meist auf der Mülldeponie, wo sie sich nur sehr langsam zersetzen können. Meistens werden sie anschließend verbrannt, was zu großen Problemen führt, da diese in den Filtern verkieseln und dadurch verstopfen. Der Staub fällt hierbei zusammen mit der Asche als Nebenprodukt an und muss aufwändig entsorgt werden.

Nachhaltigere Lösungen zum Siliconrecycling stellen beispielsweise die säure- oder basenkatalytische Äquilibrierung dar, wodurch cyclische Siloxane aus dem Prozess ausgeschleust werden können. Es findet dabei nur Si-O-Si-Bindungsspaltung aber keine Spaltung der Si-C-Bindungen statt. Rückstände, stark verunreinigte Kautschuke oder Harze sind hierfür jedoch nicht geeignet. Zudem ist der Rückstand aufgrund der starken basischen Reaktionsbedingungen hochgradig toxisch.

Durch hydrothermale Behandlung von Siliconkautschuken, d.h. der Reaktion von Siliconkautschuk mit Wasser bei hohen Temperaturen und Drücken, kann neben der Si-O-Si-Bindungsspaltung, die durch Äquilibrierung zugänglich ist, auch die Si-C-Bindung unter Bildung von Kohlenwasserstoff und Silica selektiv gespalten werden. Dieser Prozess ist allerdings sehr langsam und ist deshalb für eine Übertragung in einen technischen Prozess nicht geeignet.

Gegenstand der Erfindung ist ein Verfahren, bei dem Silicon in Gegenwart einer Base mit hydrothermalem Wasser von mindestens 200°C oder überkritischem Wasser umgesetzt wird zu Kieselsäure und Kohlenwasserstoff.

Als hydrothermal wird in diesem Zusammenhang nicht überkritisches Wasser bei Temperaturen oberhalb 200°C bezeichnet, das durch Druck größer oder gleich dem der Temperatur zugehörigen Dampfdruck im flüssigen Zustand vorliegt. Das Wasser ist aufgrund der herrschenden Druckverhältnisse noch bei weit über 100°C flüssig. Als überkritisch wird Wasser bei Temperaturen und Drücken höher oder gleich des kritischen Punktes bei 374,12°C und 22,1 MPa bezeichnet.

Überraschenderweise ist es gelungen, Si-C und Si-O Bindungen in Siliconen durch hydrothermale Umsetzung oder Umsetzung mit überkritischem Wasser zu spalten und Kohlenwasserstoffe, insbesondere Methan, sowie Kieselsäure herzustellen.
Durch die Base wird dieser Prozess beschleunigt und die vollständige Spaltung des Silicons innerhalb weniger Stunden erreicht. Zusätzlich wird durch die Base die amorphe Silica in kristalline Silica überführt. Diese Produkte können erneut im Sinne der Zirkulärwirtschaft in den Siliconherstellungsprozess eingespeist werden. Je nach Menge der zugesetzten Base ist es zudem möglich aus der durch Umsetzung des Silicons gebildeter Kieselsäure und gegebenenfalls als Füllstoff vorhandener Kieselsäure Wasserglas (Alkylsilicate) zu bilden, welches als Additiv in vielen Bereichen (z.B. Bauindustrie) genutzt werden kann.

Der Begriff Silicon umfasst oligomere oder polymere Organosiloxane, in denen Siliciumatome über Sauerstoffatome verbunden sind und in denen mindestens ein Teil der Siliciumatome einen oder mehrere organische Substituenten tragen, sowie Organosiloxane enthaltende Zusammensetzungen, wie Siliconkautschuk.

Die Silicone enthalten vorzugsweise höchstens 1 Gew.-%, besonders bevorzugt höchstens 0,1 Gew.-%, insbesondere höchstens 0,01 Gew.-% an Halogenen, insbesondere Chlor.

Organosiloxane enthaltende Zusammensetzungen können neben Organosiloxanen beispielsweise Füllstoffe, Katalysatoren, Bindemittel und Pigmente enthalten. Füllstoffe sind beispielsweise pyrogene und/oder gefällte Kieselsäure, Siliconharz, Kreide und Quartz.

Der Einsatz von Kieselsäure oder Siliconharz gefülltem Siliconkautschuk ist bevorzugt. Besonders bevorzugt wird Siliconkautschuk eingesetzt, der Füllstoff enthält, welcher ausgewählt wird aus Kieselsäure und Siliconharz.

Beispiele für geeignete Basen sind Alkali- und Erdalkalihydroxide, wie LiOH, NaOH, KOH, RbOH, CsOH, Mg(OH)₂, Ca(OH)₂, Sr(OH)₂, Ba(OH)₂, Alkali- und Erdalkalicarbonate, wie Li₂CO₃, Na₂CO₃, K₂CO₃, Alkali- und Erdalkalihydrogencarbonate, wie LiHCO₃, NaHCO₃, KHCO₃, Alkali- und Erdalkaliphosphate, wie Li₃PO₄, Na₃PO₄, K₃PO₄, Ca₃(PO₄)₂, Amine, wie Ethylendiamin, Diethylentriamin, Amide, wie Natrium- und Kaliumamid. Bevorzugt sind anorganische Basen, insbesondere Alkalihydroxide, Alkalicarbonate und Alkalihydrogencarbonate.

Das Verfahren kann diskontinuierlich oder kontinuierlich durchgeführt werden.

Das beim Verfahren anfallende Feststoffgemisch enthält vorzugsweise Kieselsäure Modifikationen, die ausgewählt werden aus Quartz, Keatit und Cristobalit.

Das beim Verfahren anfallende Feststoffgemisch enthält vorzugsweise höchstens 2 Gew.-%, besonders bevorzugt höchstens 1 Gew.-%, insbesondere höchstens 0,5 Gew.-% Kohlenstoff.

Im Falle von methylhaltigen Siliconen enthält das entstehende Gasgemisch als Kohlenwasserstoff Methan. Pro kg eingesetztem Silicon können bis zu 550 g Methan hergestellt werden. Silicone mit Phenylresten oder höheren Alkylresten bilden während der Zersetzung Benzol oder die entsprechenden Alkane, welche dann als organische Phase neben der Wasserphase vorliegen.

Die bevorzugte Temperatur im Verfahren beträgt 250°C bis 500°C, insbesondere 280°C bis 400°C und besonders bevorzugt 300°C - 390°C. Der bevorzugte Druck im Verfahren beträgt von 10 bis 400 bar, besonders bevorzugt 20 bis 320 bar, insbesondere 50 bis 280 bar, wobei als untere Grenze des Drucks jeweils der zur Reaktionstemperatur zugehörige Dampfdruck der Reaktionsmischung gilt. Die bevorzugte Verweilzeit im Verfahren beträgt 1 Minute bis 48 Stunden, besonders bevorzugt 5 Minuten bis 24 Stunden, insbesondere 10 Minuten bis 12 Stunden.

In einer bevorzugten Ausführungsform wird das Reaktionsgemisch nach Beendigung der Umsetzung auf atmosphärischen Druck entspannt und der entstehende Dampf zur Wärmerückgewinnung bzw. Vorwärmung der Edukte genutzt. Dadurch kann die Wärme zum großen Teil zurückgewonnen werden.

Folgende analytische Methoden und Geräte werden zur Charakterisierung eingesetzt:

### Beispiele

### Gaschromatografie für die Bestimmung der Zusammensetzung der Gasphase

Die Analyse der Gasphase erfolgt an einem Gaschromatographen der Marke Agilent 6890 GC unter Verwendung der Säule CP-Molsieb 5 Å mit den Dimensionen 25 m, 0.32 mm und 30 µm. Die Auswertung der Gaszusammensetzung erfolgt durch Integration der detektierten Signale.

### NMR-Spektroskopie für die Bestimmung der Silicium-haltigen Komponenten in der Wasserphase

Die Messung von ¹H-NMR und ²⁹Si-NMR Spektren erfolgt in dmso-d6 oder D2O an einem Bruker Avance 500 oder Ascend 500 (500 MHz für ¹H-NMR Spektren und 99.4 MHz für ²⁹Si-NMR Spektren). Alle Messungen sind gegen TMS als externen Standard referenziert. Die Bestimmung der relativen Verhältnisse der Bestandteile in der Wasserphase erfolgt durch Integration der jeweiligen Signalsätze. Die Bestimmung der absoluten Konzentration in Wasser erfolgt unter Berücksichtigung der Mischungsverhältnisse Wasserphase zu dmso-d6.

### Analyse des isolierten Feststoffes

### CHN-Analyse

Die Bestimmung von Sauerstoff und Wasserstoff wird am einem ONH836 Elementaranalysator durchgeführt. Der Kohlenstoffgehalt wird an einem CS844 Elementaranalysator bestimmt. Für die Analyse werden 10-20 mg Probe benötigt und unter Energiezufuhr aufgeschlossen. Wasserstoff wird anschließend in Form von Wasser, Sauerstoff in Form von CO und CO₂ mittels IR Absorption bestimmt. Kohlenstoff wird zunächst reduziert, anschließend unter Sauerstoffatmosphäre zu CO und CO2 umgesetzt und mittel IR Absorption nachgewiesen. Die Kalibrierung erfolgt gegenüber SiO₂ für Sauerstoff, gegenüber TiH₂ für Wasserstoff und gegenüber Acetanilid, Natriumhydrogencarbonat und Calciumcarbonat für Kohlenstoff.

### XRD-Analyse

Für die Bestimmung der Zusammensetzung der kristallinen Bestandteile des Feststoffes werden 5 g Pulver gemörsert und anschließend an einem Pulver Röntgen-Diffraktometer des Typs PANalytical Empyrean analysiert. Die Analyse erfolgt durch Röntgenbeugung mit Kupfer Strahlung (I = 1.54 Å).

### Reaktionsablauf:

Alle Versuche wurden in einem Inconel^{®} Druckautoklaven mit Temperatursensor und Drucksensor durchgeführt. Der Reaktionsverlauf wurde mittels Druck-Temperatur-Kurven aufgezeichnet und die Reaktionsprodukte nach Reaktionsende analysiert.

Der Siliconkautschuk wurde in Form von Würfeln mit einer Kantenlänge 0,5 cm eingesetzt.

### Beispiel 1 (nicht erfindungsgemäß):

Es wurden 24 g Siliconkautschuk (Polydimethylsiloxankautschuk WACKER ELASTOSIL^{®} 401/60E) und 100 ml Wasser eingewogen und für 12 h auf 374 °C erhitzt. Anschließend wurde der Druckautoklav auf Raumtemperatur abgekühlt. Es konnte ein Restdruck von 9 bar gemessen werden. Die Analyse zeigte die Bildung von Methan. Nach Entfernen des Überdrucks wurde der Feststoff von der wässrigen Phase getrennt und getrocknet. Es konnten 18,1 g Feststoff erhalten werden.

### Beispiel 2 (nicht erfindungsgemäß):

Es wurden 24 g Siliconkautschuk (WACKER ELASTOSIL^{®} 401/60E) und 100 ml Wasser eingewogen und bis zur Druckkonstanz (120 h) auf 374 °C erhitzt. Anschließend wurde der Druckautoklav auf Raumtemperatur abgekühlt. Es konnte ein Restdruck von 20,2 bar gemessen werden. Die Analyse zeigte die Bildung von Methan. Nach Entfernen des Überdrucks wurde der Feststoff von der wässrigen Phase getrennt und getrocknet. Es konnten 18,7 g Feststoff erhalten werden.

### Beispiel 3 (erfindungsgemäß) :

Es wurden 24 g Siliconkautschuk (WACKER ELASTOSIL^{®} 401/60E), 70 mg Kaliumhydroxid und 100 ml Wasser eingewogen und für 12 h auf 350 °C erhitzt. Ein konstanter Druck konnte bereits nach 4 h verzeichnet werden. Anschließend wurde der Druckautoklav auf Raumtemperatur abgekühlt. Es konnte ein Restdruck von 24,2 bar gemessen werden. Die Analyse zeigte die Bildung von Methan. Nach Entfernen des Überdrucks wurde der Feststoff von der wässrigen Phase getrennt und getrocknet. Es konnten 20,7 g Feststoff erhalten werden.

### Beispiel 4 (erfindungsgemäß):

Es wurden 24 g Siliconkautschuk (WACKER ELASTOSIL^{®} 401/60E), 70 mg Kaliumhydroxid und 100 ml Wasser eingewogen und für 12 h auf 300 °C erhitzt. Ein konstanter Druck konnte bereits nach ca. 5.5 h verzeichnet werden. Anschließend wurde der Druckautoklav auf Raumtemperatur abgekühlt. Es konnte ein Restdruck von 24,0 bar gemessen werden. Die Analyse zeigte die Bildung von Methan. Nach Entfernen des Überdrucks wurde der Feststoff von der wässrigen Phase getrennt und getrocknet. Es konnten 20,1 g Feststoff erhalten werden.

### Beispiel 5 (erfindungsgemäß):

Es wurden 24 g Siliconkautschuk (WACKER SilGel^{®} 612), 70 mg Kaliumhydroxid und 100 ml Wasser eingewogen und für 12 h auf 350 °C erhitzt. Ein konstanter Druck konnte bereits nach 3 h verzeichnet werden. Anschließend wurde der Druckautoklav auf Raumtemperatur abgekühlt. Es konnte ein Restdruck von 30,2 bar gemessen werden. Die Analyse zeigte die Bildung von Methan. Nach Entfernen des Überdrucks wurde der Feststoff von der wässrigen Phase getrennt und getrocknet. Es konnten 29,6 g Feststoff erhalten werden.

### Beispiel 6 (erfindungsgemäß):

Es wurden 24 g Siliconkautschuk (WACKER ELASTOSIL^{®} 401/60E), 80 mg Natriumhydroxid und 100 ml Wasser eingewogen und für 12 h auf 350 °C erhitzt. Ein konstanter Druck konnte bereits nach 1 h verzeichnet werden. Anschließend wurde der Druckautoklav auf Raumtemperatur abgekühlt. Es konnte ein Restdruck von 23,3 bar gemessen werden. Die Analyse zeigte die Bildung von Methan. Nach Entfernen des Überdrucks wurde der Feststoff von der wässrigen Phase getrennt und getrocknet. Es konnten 19,8 g Feststoff erhalten werden.

### Beispiel 7 (erfindungsgemäß):

Es wurden 24 g Siliconkautschuk (WACKER ELASTOSIL^{®} 401/60E), 80 mg Lithiumhydroxid und 100 ml Wasser eingewogen und für 12 h auf 350 °C erhitzt. Ein konstanter Druck konnte direkt nach Aufheizung verzeichnet werden. Anschließend wurde der Druckautoklav auf Raumtemperatur abgekühlt. Es konnte ein Restdruck von 24,1 bar gemessen werden. Die Analyse zeigte die Bildung von Methan. Nach Entfernen des Überdrucks wurde der Feststoff von der wässrigen Phase getrennt und getrocknet. Es konnten 18,9 g Feststoff erhalten werden.

### Beispiel 8 (erfindungsgemäß):

Es wurden 24 g Siliconkautschuk (WACKER ELASTOSIL^{®} 401/60E), 80 mg Natriumhydrogencarbonat und 100 ml Wasser eingewogen und für 12 h auf 350 °C erhitzt. Ein konstanter Druck konnte nach 5 h verzeichnet werden. Anschließend wurde der Druckautoklav auf Raumtemperatur abgekühlt. Es konnte ein Restdruck von 24,1 bar gemessen werden. Die Analyse zeigte die Bildung von Methan. Nach Entfernen des Überdrucks wurde der Feststoff von der wässrigen Phase getrennt und getrocknet. Es konnten 19,7 g Feststoff erhalten werden.

### Beispiel 9 (erfindungsgemäß):

Es wurden 24 g phenylhaltiger Siliconkautschuk (WACKER ELASTOSIL^{®} 490/55 OH), 80 mg KOH und 100 ml Wasser eingewogen und für 12 h auf 350 °C erhitzt. Ein konstanter Druck konnte nach ca. 4 h detektiert werden. Anschließend wurde der Druckautoklav auf Raumtemperatur abgekühlt und ein Restdruck von 22,3 bar gemessen. Die Analyse des Gases zeigte die Bildung von Methan. Nach Entfernen des Überdrucks konnte der Feststoff von der Flüssigkeit separiert und getrocknet werden. Es wurden 18,3 g Feststoff isoliert. Die Flüssigkeit zeigte ein Zweiphasengemisch bestehend aus einer wässrigen Phase und einer Benzolphase (ca. 1 ml), welche getrennt und mittels NMR-Spektroskopie identifiziert wurden.

### Beispiel 10 (erfindungsgemäß):

Es wurden 24 g Siliconkautschuk (WACKER ELASTOSIL^{®} 401/60E), 10 g Natriumhydroxid und 100 ml Wasser eingewogen und für 12 h auf 350 °C erhitzt. Ein konstanter Druck konnte direkt nach Aufheizung verzeichnet werden. Anschließend wurde der Druckautoklav auf Raumtemperatur abgekühlt. Es konnte ein Restdruck von 23,1 bar gemessen werden. Die Analyse zeigte die Bildung von Methan. Nach Entfernen des Überdrucks wurde eine wässrige Lösung isoliert. Der Feststoffgehalt der Wasserphase beträgt 28,5%. Das entstandene Natriumsilikat kann durch Trocknung der wässrigen Lösung isoliert werden und zeigt einen Restkohlenstoffgehalt von 0.07%.

**Tabelle 1**

| Beispiel | Wässrige Phase: | Feststoff: | |
|---|---|---|---|
| | m(Silanole) | EA (C in %) | XRD |
| 1* | 870 mg | 14,2 | -^{‡} |
| 2* | 170 mg | 3, 4 | -^{‡} |
| 3 | 14 mg | 0,10 | 52,2% Quartz, 22,8% Cristobalit 24% Keatit |
| 4 | 68 mg | 0,13 | 100% Keatit |
| 5 | 30 mg | 0,14 | 29,4% Quartz, 70,6% Keatit |
| 6 | 17 mg | 0,03 | 98,5% Quartz, 1,5% Keatit |
| 7 | 32 mg | 0,11 | 100% Quartz |
| 8 | 48 mg | 0,10 | 31% Quartz, 69% Keatit |
| 9 | 15 mg | 0,12 | 42% Quarz, 33,2% Cristobalit, 24,8% Keatit |
| 10 | - | - | - |

| | | | |
|---|---|---|---|
| * nicht erfindungsgemäß * Probe ist amorph | | | |

## Patentansprüche

1. Verfahren, bei dem Silicon in Gegenwart einer Base mit hydrothermalem Wasser von mindestens 200°C oder überkritischem Wasser umgesetzt wird zu Kieselsäure und Kohlenwasserstoff.

2. Verfahren nach Anspruch 1 bei dem der Begriff Silicon oligomere oder polymere Organosiloxane umfasst, in denen Siliciumatome über Sauerstoffatome verbunden sind und in denen mindestens ein Teil der Siliciumatome einen oder mehrere organische Substituenten tragen, sowie Organosiloxane enthaltende Zusammensetzungen.

3. Verfahren nach einem oder mehreren der voranstehenden Ansprüche, bei dem der Begriff Silicon Siliconkautschuk bedeutet, der Füllstoff enthält, welcher ausgewählt wird aus Kieselsäure und Siliconharz.

4. Verfahren nach einem oder mehreren der voranstehenden Ansprüche, bei dem die Silicone höchstens 1 Gew.-% an Halogenen enthalten.

5. Verfahren nach einem oder mehreren der voranstehenden Ansprüche, bei dem die Base ausgewählt wird aus Alkali- und Erdalkalihydroxiden, Alkali- und Erdalkalicarbonaten, Alkali- und Erdalkalihydrogencarbonaten, Alkali- und Erdalkaliphosphaten, Aminen und Amiden.

6. Verfahren nach einem oder mehreren der voranstehenden Ansprüche, bei dem die Temperatur 300°C bis 400°C beträgt.

7. Verfahren nach einem oder mehreren der voranstehenden Ansprüche, bei dem der Druck 10 bis 400 bar beträgt.

8. Verfahren nach einem oder mehreren der voranstehenden Ansprüche, bei dem methylhaltiges Silicon eingesetzt wird und der gebildete Kohlenwasserstoff Methan ist.

## Claims

1. A process in which silicone is reacted in the presence of a base with hydrothermal water of at least 200°C or supercritical water to form silica and hydrocarbon.

2. The process as claimed in claim 1, in which the term silicone encompasses oligomeric or polymeric organosiloxanes in which silicon atoms are bonded via oxygen atoms and in which at least some of the silicon atoms bear one or more organic substituents, and compositions comprising organosiloxanes.

3. The process as claimed in one or more of the preceding claims, in which the term silicone means silicone rubber comprising filler which is selected from silica and silicone resin.

4. The process as claimed in one or more of the preceding claims, in which the silicones comprise at most 1% by weight of halogens.

5. The process as claimed in one or more of the preceding claims, in which the base is selected from alkali metal and alkaline earth metal hydroxides, alkali metal and alkaline earth metal carbonates, alkali metal and alkaline earth metal hydrogencarbonates, alkali metal and alkaline earth metal phosphates, amines and amides.

6. The process as claimed in one or more of the preceding claims, in which the temperature is 300°C to 400°C.

7. The process as claimed in one or more of the preceding claims, in which the pressure is 10 to 400 bar.

8. The process as claimed in one or more of the preceding claims, in which methyl-containing silicone is used and the hydrocarbon formed is methane.

## Revendications

1. Procédé dans lequel la silicone est mise à réagir en présence d'une base avec de l'eau hydrothermale d'au moins 200 °C ou de l'eau supercritique pour donner de la silice et un hydrocarbure.

2. Procédé selon la revendication 1, dans lequel le terme silicone comprend les organosiloxanes oligomères ou polymères dans lesquels les atomes de silicium sont liés par l'intermédiaire d'atomes d'oxygène et dans lesquels au moins une partie des atomes de silicium portent un ou plusieurs substituants organiques, ainsi que les compositions contenant des organosiloxanes.

3. Procédé selon une ou plusieurs des revendications précédentes, dans lequel le terme silicone désigne un caoutchouc silicone qui contient une charge choisie parmi la silice et une résine silicone.

4. Procédé selon une ou plusieurs des revendications précédentes, dans lequel les silicones contiennent au plus 1 % en poids d'halogènes.

5. Procédé selon une ou plusieurs des revendications précédentes, dans lequel la base est choisie parmi les hydroxydes de métaux alcalins et alcalino-terreux, les carbonates de métaux alcalins et alcalino-terreux, les hydrogénocarbonates de métaux alcalins et alcalino-terreux, les phosphates de métaux alcalins et alcalino-terreux, les amines et les amides.

6. Procédé selon une ou plusieurs des revendications précédentes, dans lequel la température est de 300 °C à 400 °C.

7. Procédé selon une ou plusieurs des revendications précédentes, dans lequel la pression est de 10 à 400 bars.

8. Procédé selon une ou plusieurs des revendications précédentes, dans lequel on utilise une silicone contenant du méthyle et l'hydrocarbure formé est le méthane.
